# EUROPEAN PATENT APPLICATION

(11) **EP 4 014 882 A1**
(43) Date of publication of application: **22.06.2022**
(21) Application number: 21154887.0
(22) Date of filing: 03.02.2021
(51) Int. Cl.: A61B 8/00

(54) **ULTRASONIC DIAGNOSTIC APPARATUS AND PROBE FOR THE SAME**

(30) Priority: 18.12.2020 WO PCT/CN2020/137507
(71) Applicant: Koninklijke Philips N.V., 5656 AG Eindhoven (NL)
(72) Inventor: CHEN, Xidong, 5656 AE Eindhoven (NL)
(74) Representative: Philips Intellectual Property & Standards

(57) **Abstract**

The present invention provides a probe for an ultrasonic diagnostic apparatus. The probe comprises a bulkhead, a driving mechanism supported by the bulkhead, a transducer being driven via the driving mechanism by a motor to swing, a contact portion engaging hermetically with the bulkhead to define a sealed space, and a bubble trap device which can be selectively in fluid communication with the sealed space via a passage between the sealed space and the bubble trap device. Both the sealed space and the bubble trap device are completely filled with a liquid medium, and the bubble trap device is configured to allow bubbles in the liquid medium to enter the bubble trap device from the sealed space when the probe is positioned in the downward position but prevent the bubbles from escaping from the bubble trap device when the probe is positioned in the upward position. According to the present invention, the probe performance is not deteriorated by the bubbles and an accurate diagnosis is achieved.

## Description

### FIELD OF THE INVENTION

The invention relates to an ultrasonic diagnostic apparatus, and more particularly, to a probe used for an ultrasonic diagnostic apparatus.

### BACKGROUND OF THE INVENTION

An ultrasonic diagnostic apparatus generally includes a main body including an operation portion to input various types of commands and information required for an ultrasonic diagnosis and a display portion to display an image when the ultrasonic diagnosis is performed. The ultrasonic diagnostic apparatus also includes a probe which electrically and electronically communicates with the main body and may be attached onto a subject to transmit ultrasonic waves to an inside of the subject and to receive the ultrasonic waves reflected from the subject. Since the ultrasonic waves have a different reflectivity in a boundary surface of two different materials, it is possible to obtain the internal shape of the subject by analyzing the reflected ultrasonic waves. A sectional image of a portion where the ultrasonic waves pass through may be created by reconstructing information received by the probe. The sectional image may be output on the display portion of the ultrasonic diagnostic apparatus. Through a review on the output image, a user may verify a size of the internal tissue of the subject, a shape thereof, a location thereof, a color thereof, and the like, and thereby may diagnose an internal status of the subject.

A conventional probe includes a bulkhead, a transducer supported on the bulkhead to transmit and receive ultrasonic waves, a contact portion being provided in front of the transducer to contact with a subject, and a liquid medium being filled in a space between the bulkhead and the contact portion to transfer the ultrasonic waves from the transducer to the contact portion or from the contact portion to the transducer. The liquid medium itself may contain tiny bubbles. Also, due to an unskillfulness of an operator, or due to a complex internal structure of the probe, the bubbles may occur while filling or injecting the liquid medium.

In most cases, the probe is used to be attached onto the subject with the contact portion of the probe facing downwardly when the subject is lying down. The contact portion of the probe generally faces upwardly when the ultrasonic diagnostic apparatus is on standby, performs the ultrasonic diagnosis for the subject who can not lie down or performs the ultrasonic diagnosis for a special portion of the subject (for example, the axilla). The bubbles contained in the liquid medium of the probe tend to move upwardly in the liquid medium. The bubbles contained in the liquid medium of the probe move upwardly and gather between the transducer and the contact portion of the probe when the contact portion of the probe faces upwardly or inclines upwardly. The bubbles gathering between the transducer and the contact portion of the probe may obstruct a transfer of the ultrasonic waves through the liquid medium. Accordingly, the ultrasonic waves may not be normally transferred from the transducer to the contact portion or from the contact portion to the transducer, which may result in deteriorating a probe performance and causing an inaccurate diagnosis.

Thus, there is a need to make improvements on the conventional probe for the ultrasonic diagnostic apparatus.

### SUMMARY OF THE INVENTION

According to one aspect of the present invention, it is to provide a probe for an ultrasonic diagnostic apparatus comprising:
- a bulkhead;
- a driving mechanism supported by the bulkhead;
- a transducer for transmitting and receiving ultrasonic waves, the transducer being driven via the driving mechanism by a motor to swing;
- a contact portion engaging hermetically with the bulkhead to define a sealed space;
- a bubble trap device which can be selectively in fluid communication with the sealed space via a passage between the sealed space and the bubble trap device;
- wherein both the sealed space and the bubble trap device are completely filled with a liquid medium, and
- the bubble trap device is configured to allow bubbles in the liquid medium to enter the bubble trap device from the sealed space when the probe is positioned in a downward position where the contact portion faces downwardly but prevent the bubbles from escaping from the bubble trap device when the probe is positioned in an upward position where the contact portion faces upwardly. It should be understood that the expression "the contact portion faces downwardly" refers to "an apex of the contact portion points downwardly", wherein "pointing downwardly" comprises not only "uprightly pointing downwardly" but also "obliquely pointing downwardly". Similarly, the expression "the contact portion faces upwardly" refers to "an apex of the contact portion points upwardly", wherein "pointing upwardly" comprises not only "uprightly pointing upwardly" but also "obliquely pointing upwardly"

Preferably, the bubble trap device may comprise a trap container and a floating body, the trap container can be selectively in fluid communication with the sealed space via the passage between the sealed space and the trap container, the floating body is made from a material whose density is less than that of the liquid medium so that the floating body always moves upwardly in the liquid medium, the floating body opens the passage between the sealed space and the trap container to allow the bubbles in the liquid medium to move from the sealed space into the trap container when the probe is positioned in the downward position, and the floating body blocks the passage between the sealed space and the trap container to prevent the bubbles in the liquid medium from moving from the trap container into the sealed space when the probe is positioned in the upward position.

Preferably, the trap container may comprise an inlet portion having a circular cross section and a container body connecting and communicating with the inlet portion, the floating body has a circular cross section, the largest diameter of the floating body is larger than the largest inner diameter of the inlet portion.

Preferably, the trap container may further comprise a transition portion connecting the inlet portion and the container body, the inner diameter of the transition portion is larger than the largest diameter of the floating body.

Preferably, a through hole may be formed in the top of the container body opposite to the inlet portion and is sealed by a seal member.

Preferably, the inner diameter of the inlet portion is substantially constant.

Preferably, the inner diameter of the inlet portion gradually decreases toward the sealed space.

Preferably, the container body is configured as an elongated body having a substantially right-angled trapezoidal cross section.

Preferably, a sloping side of the elongated body may limit the position of the floating body so that the floating body is substantially in alignment with the inlet portion when the probe is positioned in the downward position.

Preferably, the container body is configured as a substantially cylindrical body.

Preferably, a limiting-position portion may project from the top of the container body toward the inlet portion, the limiting-position portion limits the position of the floating body to be substantially in alignment with the inlet portion when the probe is positioned in the downward position.

Preferably, the floating body is a floating ball, a floating truncated cone or a floating cone.

Preferably, a gap is defined between the floating body and the container body when the probe is positioned in the downward position.

Preferably, a portion of the bulkhead adjacent the inlet portion is shaped as a curved guide surface to guide the bubbles to enter the trap container.

According to one aspect of the present invention, it is to provide an ultrasonic diagnostic apparatus comprising a probe as stated above.

The probe according to the present invention comprises the bubble trap device configured to allow bubbles in the liquid medium to enter the bubble trap device from the sealed space when the probe is positioned in the downward position but prevent the bubbles from escaping from the bubble trap device when the probe is positioned in the upward position. No bubble gathers between the transducer and the contact portion of the probe. Accordingly, the ultrasonic waves may be normally transferred from the transducer to the contact portion or from the contact portion to the transducer. As a result, the probe performance is not deteriorated by the bubbles and an accurate diagnosis is achieved.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
Fig. 1 is a side view of a potion of a probe according to an embodiment of the present invention;
Fig. 2 is a top view illustrating the potion of the probe of Fig. 1;
Fig. 3 is a cross-sectional view taken along a line 3-3 of Fig. 2;
Fig. 4 is a cross-sectional view taken along a line 4-4 of Fig. 2;
Fig. 5 is a perspective view illustrating a bubble trap device according to a first embodiment of the present invention;
Fig. 6 is a top view illustrating the bubble trap device of Fig. 5;
Fig. 7 is a rear view illustrating the bubble trap device of Fig. 5;
Fig. 8 is a cross-sectional view taken along a line 8-8 of Fig. 7;
Fig. 9 is a cross-sectional view similar to Fig. 3, illustrating the bubbles moving upwardly into the bubble trap device;
Fig. 10 is a cross-sectional view similar to Fig. 3 but with the contact portion facing upwardly, illustrating the bubbles trapped in the bubble trap device;
Fig. 11 is a front view illustrating a bubble trap device according to a second embodiment of the present invention;
Fig. 12 is a cross-sectional view taken along a line 12-12 of Fig. 11;
Fig. 13 is a front view illustrating a bubble trap device according to a third embodiment of the present invention;
Fig. 14 is a cross-sectional view taken along a line 14-14 of Fig. 13;
Fig. 15 is a front view illustrating a bubble trap device according to a fourth embodiment of the present invention; and
Fig. 16 is a cross-sectional view taken along a line 16-16 of Fig. 15.

### DETAILED DESCRIPTION OF THE EXEMPLARY EMBODIMENTS

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

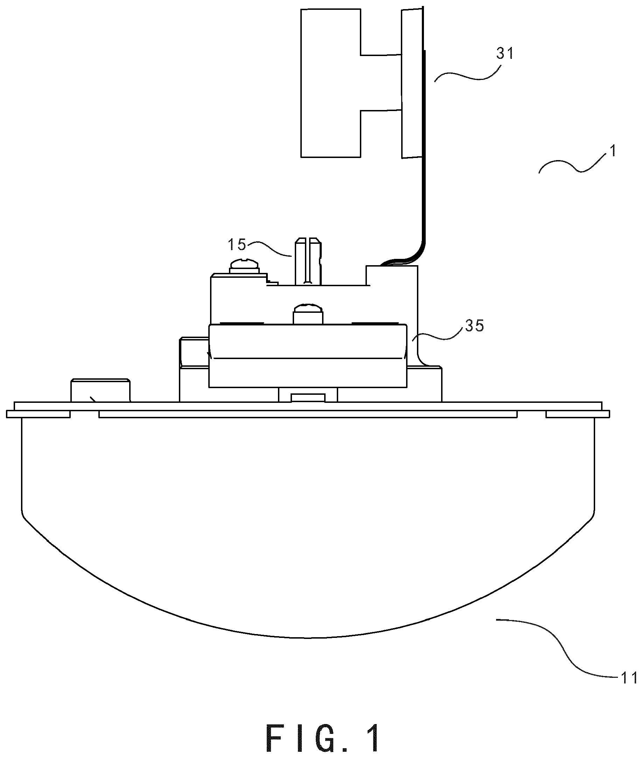

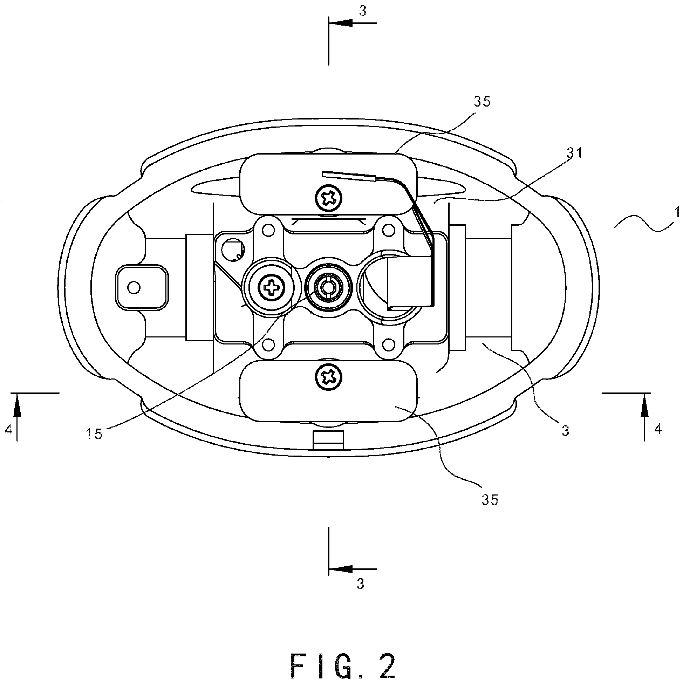

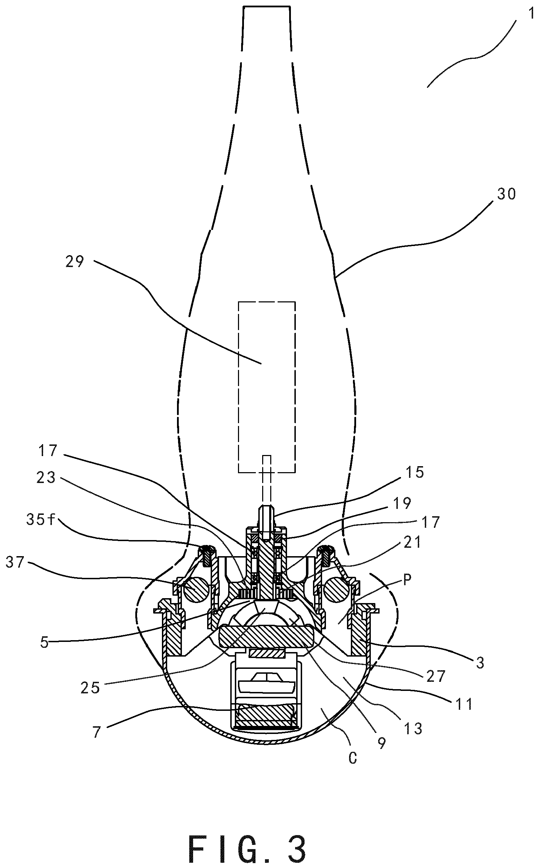

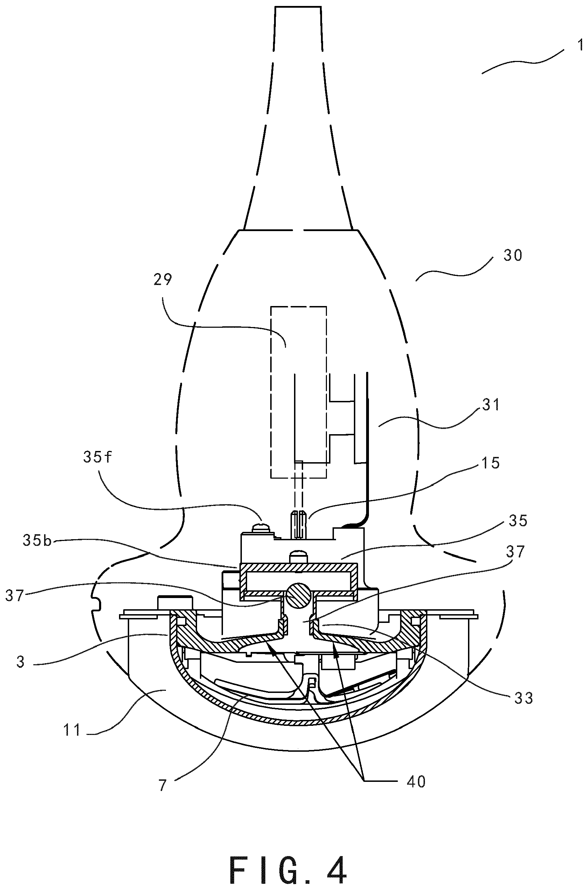

Fig. 1 is a side view of a potion of a probe according to an embodiment of the present invention, Fig.. 2 is a top view illustrating the potion of the probe of Fig. 1, Fig. 3 is a cross-sectional view taken along a line 3-3 of Fig. 2 and Fig. 4 is a cross-sectional view taken along a line 4-4 of Fig. 2.

Referring to Figs. 1 through 4, a probe 1 according to an embodiment of the present invention may include a bulkhead 3, a driving mechanism 5 supported by the bulkhead 3, a transducer 7 mounted onto a holder 9, and a contact portion (also called "cover") 11. The contact portion 11 engages hermetically with the bulkhead 3 to define a sealed space C in which a liquid medium 13 is completely filled and the transducer 7 can swing in the liquid medium 13.

The driving mechanism 5 includes a drive shaft 15 supported rotatably on bulkhead 3 by means of bearings 17 and a rotary seal 19. The drive shaft 15 is provided with a small gear 21 at one end extending towards the transducer 7. The small gear 21 meshes with a large gear 23 on which a small bevel gear 25 is formed integrally. The small bevel gear 25 meshes with a large bevel half gear 27 with which the holder 9 couples fixedly. The drive shaft 15 is connected at the other end with an output shaft of a motor 29 contained in a housing 30. When the motor 29 rotates, the driving mechanism 5 converts the rotary motion of the motor 29 into the reciprocating swing motion of the transducer 7 in the liquid medium 13 to perform the ultrasonic diagnosis using an ultrasonic diagnostic apparatus. It should be understood that the driving mechanism 5 can be implemented in any suitable ways else.

The liquid medium 13 may be a flowing medium material that may transfer the ultrasonic waves from the transducer 7 to the contact portion 11 or from the contact portion 11 to the transducer 7. The liquid medium 13 may be completely filled in the sealed space C formed between the bulkhead 3 and the contact portion 11. Oil or saline solution may be used as the liquid medium 13. Hereinafter, the present invention will be described based on a case where the oil is used for the liquid medium 13.

The contact portion 11 may transfer the ultrasonic waves between the subject and the liquid medium 13. When the ultrasonic diagnosis is performed using the ultrasonic diagnostic apparatus, the contact portion 11 contacts with the subject. The contact portion 11 may be convexly provided in a form of a dome corresponding to the transducer 7.

The transducer 7 functions to transmit ultrasonic waves to an inside of the subject and to receive the ultrasonic waves reflected from the subject when the ultrasonic diagnosis is performed using the ultrasonic diagnostic apparatus. The ultrasonic waves received by the transducer 7 are converted into electrical signal which is transferred to a main body (not shown) of the ultrasonic diagnostic apparatus via a flexible circuit 31. The main body processes and reconstructs the electrical signal so that a sectional image of a portion where the ultrasonic waves pass through may be created and displayed on a display portion (not shown) of the ultrasonic diagnostic apparatus.

Bubbles B within the liquid medium 13 may be dispersed in the liquid medium 13 and/or adhere to the internal components, for example the transducer or the driving mechanism, of the probe 1. When the probe 1 operates, the bubbles B may move upwardly in the liquid medium 13 due to a weight difference between the liquid medium 13 and the bubbles B, or due to the vibration of the transducer 7. The upwardly moved bubbles B may gather between the transducer and the contact portion of the probe when the contact portion of the probe faces upwardly or inclines upwardly. The bubbles gathering between the transducer and the contact portion of the probe may obstruct a transfer of the ultrasonic waves between the contact portion and the liquid medium.

The probe 1 according to an embodiment of the present invention may further include a bubble trap device 33. The bubble trap device 33 may comprise a trap container 35 in fluid communication with the sealed space C of the probe 1 and thus is completely filled with the liquid medium 13. The bubble trap device 33 may further comprise a floating body 37 for opening or blocking a passage P between the trap container 35 and the sealed space C. The bubble trap device 33 thus is configured to allow the bubbles B in the liquid medium 13 to enter bubble trap device 33 from the sealed space C when the contact portion 11 faces downwardly but prevent the bubbles B from escaping from bubble trap device 33 when the contact portion 11 faces upwardly.

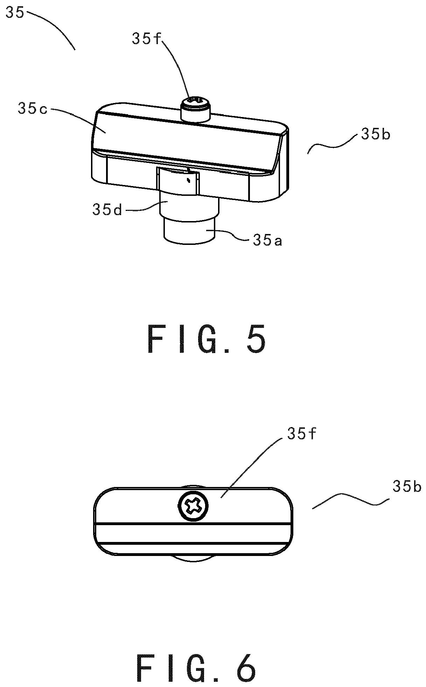

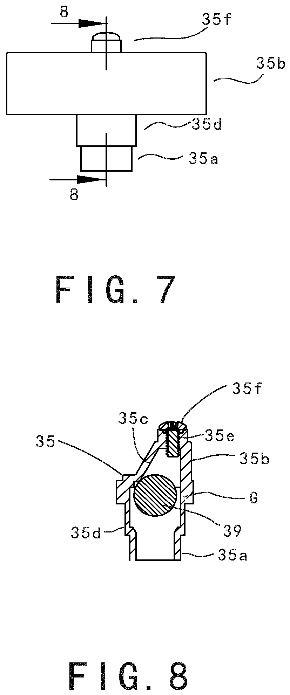

Fig. 5 is a perspective view illustrating a bubble trap device according to a first embodiment of the present invention, Fig. 6 is a top view illustrating the bubble trap device of Fig. 5, Fig. 7 is a rear view illustrating the bubble trap device of Fig. 5 and Fig. 8 is a cross-sectional view taken along a line 8-8 of Fig. 7. Referring to Figs. 5 through 8, the trap container 35 comprises an inlet portion 35a having a circular cross section and a container body 35b connecting and communicating with the inlet portion 35a. The inner diameter of the inlet portion 35a is substantially constant. The floating body 37 comprises a floating ball 39 which always floats upwardly in the liquid medium 13 and has a diameter larger than an inner diameter of the inlet portion 35a. Although the floating ball 39 may be a hollow ball, it is preferably a solid ball to prevent from bursting when the sealed space C is filled with the liquid medium 13 under vacuum. The floating ball 39 is made from a material such as polyethylene having a density which is less than that of the liquid medium 13.

In the preferred embodiments as shown in Fig. 5 through 8, the container body 35b is an elongated body having a substantially right-angled trapezoidal cross section. A sloping side 35c of the container body 35b may limit the position of the floating ball 39 in the container body 35b so that the floating ball 39 is substantially in alignment with the inlet portion 35a when the contact portion 11 faces downwardly. Such alignment facilitates the floating ball 39 to reliably move to a position in the passage P between the trap container 35 and the sealed space C is blocked by the floating ball 39 when the contact portion 11 faces upwardly. Preferably, the trap container 35 may further comprise a transition portion 35d connecting the inlet portion 35a and the container body 35b. The transition portion 35d also has a circular cross section and has an inner diameter which is larger than the inner diameter of the inlet portion 35a and the diameter of the floating ball 39. As a result, the floating ball 39 is at least partially located in the transition portion 35d while a gap G exists between the floating ball 39 and the transition portion 35d.

A through hole 35e is formed in the top of the container body 35b opposite to the inlet portion 35a. In the present invention, "the top of the container body" means a relative orientation when the probe is positioned in the downward position, as shown in Figs 3 and 4. The through hole 35e may be sealed by a seal member 35f such as a seal screw or a seal plug. The through hole 35e may discharge the air from the sealed space C and the trap container 35 when the sealed space C and the trap container 35 are filled with the liquid medium 13 under vacuum. Once the sealed space C and the trap container 35 are completely filled with the liquid medium 13, the through hole 35e is sealed by the seal member 35f.

The trap container 35 is mounted onto the bulkhead 3 at a side opposite to the transducer 7, for example by sealingly inserting the inlet portion 35a in a hole formed in the bulkhead 3. Thus, the passage P is defined between the trap container 35 and the sealed space C so that the trap container 35 is in fluid communication with the sealed space C through the passage P while the container body 35b is received in the housing 30. To have the largest volume without substantially changing the structure of the probe, the container body 35b is configured as an elongated body having a substantially right-angled trapezoidal cross section to make use of a space between the bulkhead 3 and the housing 30.

In order to guide the bubbles B to enter the trap container 35, a portion of the bulkhead 3 adjacent the inlet portion 35a is shaped as a curved guide surface 40. In addition, the number of the bubble trap device may be one or more than two although two bubble trap devices are shown to position at either side of the transducer 7 in the illustrated embodiment. Further, the trap container 35 may comprise two separate portions which can be assembled together so that the floating ball 39 may be easily disposed within the trap container 35.

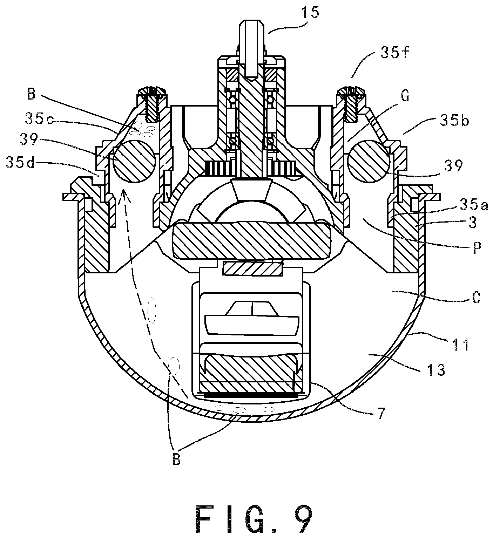

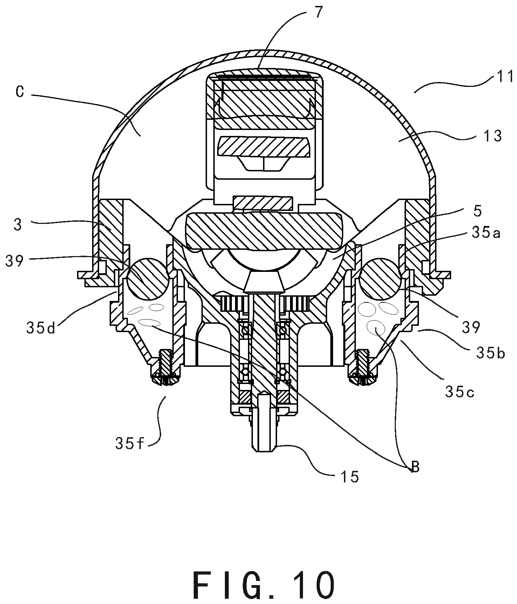

Operation of the bubble trap device 33 is described below with reference to Figs. 9 and 10. Fig. 9 is a cross-sectional view similar to Fig. 3, illustrating the bubbles moving upwardly into the bubble trap device. Fig. 10 is a cross-sectional view similar to Fig. 3 but with the contact portion facing upwardly, illustrating the bubbles trapped in the bubble trap device. When the probe 1 is in a state in which the contact portion 11 faces downwardly, the floating ball 39 floats upwardly in the liquid medium 13 until its movement is limited by the sloping side 35c of the container body 35b. Since the inner diameter of the transition portion 35d is larger than the diameter of the floating ball 39, the gap G is defined between the floating ball 39 and the transition portion 35d. As a result, the bubbles B in the liquid medium 13 within the sealed space C move upwardly through the gap G into the container body 35b and gather over the floating ball 39, as shown in Fig. 9. When the probe 1 is in a state in which the contact portion 11 faces upwardly, the floating ball 39 also floats upwardly in the liquid medium 13 until its movement is limited by the inlet portion 35a. Since the inner diameter of the inlet portion 35a is smaller than the diameter of the floating ball 39, the opening of the inlet portion 35a is blocked by the floating ball 39. As a result, it is possible to prevent the bubbles B in the liquid medium 13 within the container body 35b from moving upwardly into the sealed space C, as shown in Fig. 10. In this way, the bubbles B in the liquid medium 13 can only enter the trap container 35 from the sealed space C when the contact portion 11 faces downwardly but is prevented from escaping from the trap container 35 when the contact portion 11 faces upwardly.

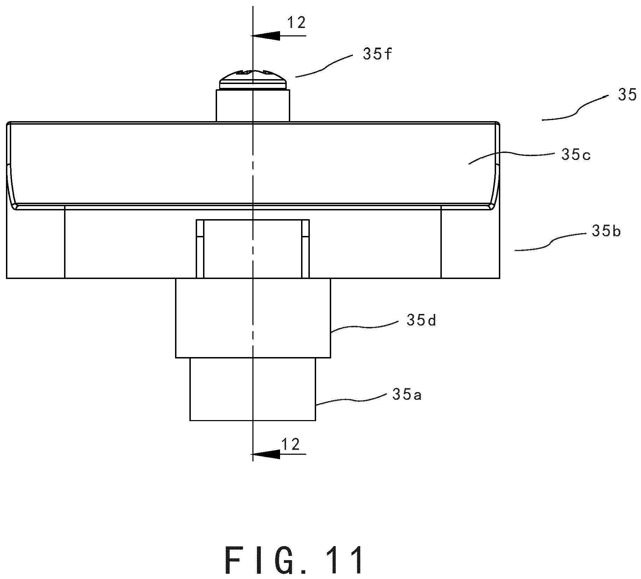

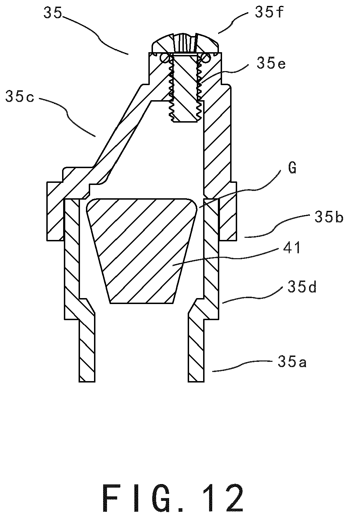

Fig. 11 is a front view illustrating a bubble trap device according to a second embodiment of the present invention and Fig. 12 is a cross-sectional view taken along a line 12-12 of Fig. 11. The structure of the bubble trap device according to the second embodiment of the present invention is substantially similar to that of the bubble trap device according to the first embodiment of the present invention. For the sake of simplicity and easy understanding of the invention, a description common to the first embodiment is omitted. In the second embodiment, the floating body 37 comprises a floating truncated cone 41 instead of the floating ball 39. It should be understood that a floating cone is also feasible. The floating truncated cone 41 more easily aligns with the inlet portion 35a and thus more reliably blocks the opening of the inlet portion 35a.

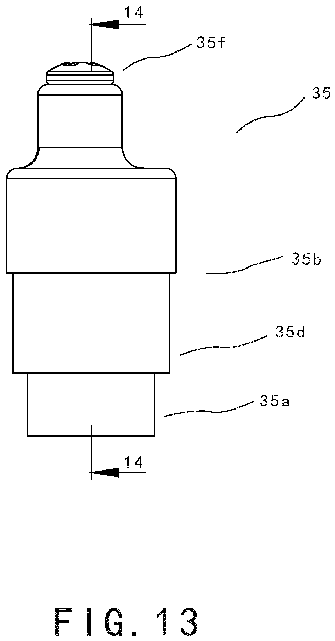

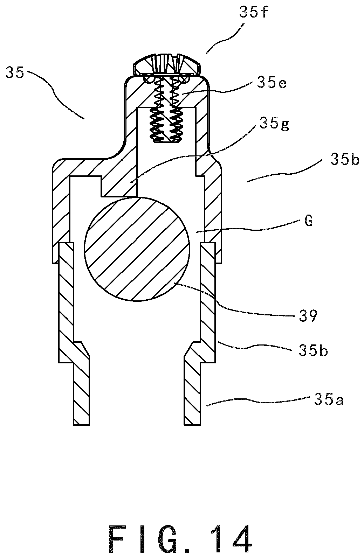

Fig. 13 is a front view illustrating a bubble trap device according to a third embodiment of the present invention and Fig. 14 is a cross-sectional view taken along a line 14-14 of Fig. 13. The structure of the bubble trap device according to the third embodiment of the present invention is substantially similar to that of the bubble trap device according to the first embodiment of the present invention. For the sake of simplicity and easy understanding of the invention, a description common to the first embodiment is omitted. In the third embodiment, the container body 35b is a substantially cylindrical body instead of the elongated body having a substantially right-angled trapezoidal cross section. A limiting-position portion 35g may project from the top of the container body 35b toward the inlet portion 35a. The limiting-position portion 35g may limit the position of the floating ball 39 to be substantially in alignment with the inlet portion 35a so that the floating ball 39 may quickly move toward the inlet portion 35a and block the opening of the inlet portion 35a when the probe 1 is in a state in which the contact portion 11 faces upwardly.

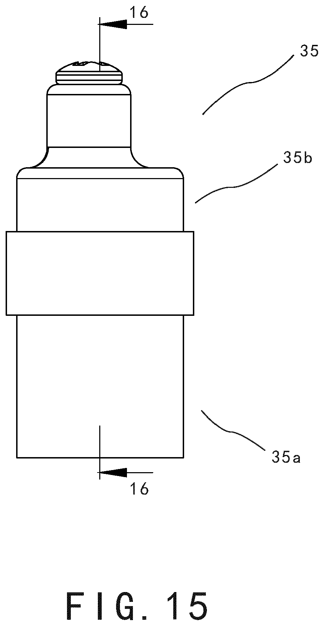

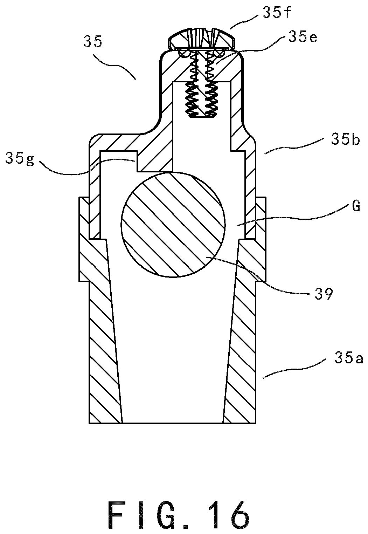

Fig. 15 is a front view illustrating a bubble trap device according to a fourth embodiment of the present invention and Fig. 16 is a cross-sectional view taken along a line 16-16 of Fig. 15. The structure of the bubble trap device according to the fourth embodiment of the present invention is substantially similar to that of the bubble trap device according to the third embodiment of the present invention. For the sake of simplicity and easy understanding of the invention, a description common to the third embodiment is omitted. Other than the inlet portion 35a having a substantially constant inner diameter as shown in Figs. 13 and 14, the inner diameter of the inlet portion 35a according to the fourth embodiment gradually decreases toward the sealed space C. The inlet portion 35a according to the fourth embodiment may guide the floating ball 39 to quickly move and block the opening of the inlet portion 35a when the probe 1 is in a state in which the contact portion 11 faces upwardly.

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims.

## Claims

1. A probe (1) for an ultrasonic diagnostic apparatus comprising:
- a bulkhead (3);
- a driving mechanism (5) supported by the bulkhead (3);
- a transducer (7) for transmitting and receiving ultrasonic waves, the transducer (7) being driven via the driving mechanism (5) by a motor (29) to swing;
- a contact portion (11) engaging hermetically with the bulkhead (3) to define a sealed space (C);
- a bubble trap device (33) which is selectivly in fluid communication with the sealed space (C) via a passage (P) between the sealed space (C) and the bubble trap device (33) ;
- wherein both the sealed space (C) and the bubble trap device (33) are completely filled with a liquid medium (13), and
- the bubble trap device (33) is configured to allow bubbles (B) in the liquid medium (13) to enter the bubble trap device (33) from the sealed space (C) when the probe (1) is positioned in a downward position where the contact portion (11) faces downwardly but prevent the bubbles (B) from escaping from the bubble trap device (33) when the probe (1) is positioned in an upward position where the contact portion (11) faces upwardly.

2. The probe (1) for an ultrasonic diagnostic apparatus according to claim 1, wherein the bubble trap device (33) comprises a trap container (35) and a floating body (37), the trap container (35) is selectively in fluid communication with the sealed space (C) via the passage (P) between the sealed space (C) and the trap container (35), the floating body (37) is made from a material whose density is less than that of the liquid medium (13) so that the floating body (37) moves upwardly in the liquid medium (13), the floating body (37) opens the passage (P) between the sealed space (C) and the trap container (35) to allow the bubbles (B) in the liquid medium (13) to move from the sealed space (C) into the trap container (35) when the probe (1) is positioned in the downward position, and the floating body (37) blocks the passage (P) between the sealed space (C) and the trap container (35) to prevent the bubbles (B) in the liquid medium (13) from moving from the trap container (35) into the sealed space (C) when the probe (1) is positioned in the upward position.

3. The probe (1) for an ultrasonic diagnostic apparatus according to claim 2, wherein the trap container (35) comprises an inlet portion (35a) having a circular cross section and a container body (35b) connecting and communicating with the inlet portion (35a), the floating body (37) has a circular cross section, the largest diameter of the floating body (37) is larger than the largest inner diameter of the inlet portion (35a).

4. The probe (1) for an ultrasonic diagnostic apparatus according to claim 3, wherein the trap container (35) further comprises a transition portion (35d) connecting the inlet portion (35a) and the container body (35b), the inner diameter of the transition portion (35d) is larger than the largest diameter of the floating body (37).

5. The probe (1) for an ultrasonic diagnostic apparatus according to claim 3, wherein a through hole (35e) is formed in the top of the container body (35b) opposite to the inlet portion (35a) and is sealed by a seal member (35f).

6. The probe (1) for an ultrasonic diagnostic apparatus according to claim 3, wherein the inner diameter of the inlet portion (35a) is substantially constant.

7. The probe (1) for an ultrasonic diagnostic apparatus according to claim 3, wherein the inner diameter of the inlet portion (35a) gradually decreases toward the sealed space (C).

8. The probe (1) for an ultrasonic diagnostic apparatus according to claim 3, wherein the container body (35b) is configured as an elongated body having a substantially right-angled trapezoidal cross section.

9. The probe (1) for an ultrasonic diagnostic apparatus according to claim 8, wherein a sloping side (35c) of the elongated body limits the position of the floating body (37) so that the floating body (37) is substantially in alignment with the inlet portion (35a) when the probe (1) is positioned in the downward position.

10. The probe (1) for an ultrasonic diagnostic apparatus according to claim 3, wherein the container body (35b) is configured as a substantially cylindrical body.

11. The probe (1) for an ultrasonic diagnostic apparatus according to claim 10, wherein a limiting-position portion (35g) projects from the top of the container body (35b) toward the inlet portion (35a), the limiting-position portion (35g) limits the position of the floating body (37) to be substantially in alignment with the inlet portion (35a) when the probe (1) is positioned in the downward position.

12. The probe (1) for an ultrasonic diagnostic apparatus according to claim 2, wherein the floating body (37) is a floating ball (39), a floating truncated cone (41) or a floating cone.

13. The probe (1) for an ultrasonic diagnostic apparatus according to claim 3, wherein a gap (G) is defined between the floating body (37) and the container body (35b) when the probe (1) is positioned in the downward position.

14. The probe (1) for an ultrasonic diagnostic apparatus according to claim 3, wherein a portion of the bulkhead (3) adjacent the inlet portion (35a) is shaped as a curved guide surface (40) to guide the bubbles (B) to enter the trap container (35).

15. An ultrasonic diagnostic apparatus comprising a probe (1) according to any one of claims 1-14.
